# EUROPEAN PATENT APPLICATION

(11) **EP 3 533 448 A1**
(43) Date of publication of application: **04.09.2019**
(21) Application number: 17864986.9
(22) Date of filing: 25.10.2017
(51) Int. Cl.: A61K 31/497, A61K 9/20, A61K 47/02, A61P 35/00

(54) **STABLE PHARMACEUTICAL COMPOSITION**

(30) Priority: 26.10.2016 JP 2016209127
(71) Applicant: Astellas Pharma Inc., Chuo-ku Tokyo 103-8411 (JP)
(72) Inventor: SAKURAI, Atsushi, Tokyo 103-8411 (JP); YOSHIDA, Mitsuru, Tokyo 103-8411 (JP); TOMINAGA, Tetsuo, Tokyo 103-8411 (JP)
(74) Representative: Hoffmann Eitle
(86) International application number: PCT/JP2017/038416
(87) International publication number: WO 2018/079570

(57) **Abstract**

Provided is a pharmaceutical composition, which comprises 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide or a pharmaceutically acceptable salt thereof, and is stabilized. The pharmaceutical composition comprises potassium chloride and/or sodium chloride, and inhibits the increase of related substances.

## Description

### TECHNICAL FIELD

The present invention relates to a stable pharmaceutical composition comprising 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide or a pharmaceutically acceptable salt thereof, and potassium chloride and/or sodium chloride.

### BACKGROUND ART

5-[(3R)-1-Acryloylpyrrolidin-3-yl]oxy}-6-ethyl-3-({4-[4-(4-methylpiperazin-1-yl)piperidin-1-yl]phenyl}amino)-pyrazine-2-carboxamide (hereinafter sometimes referred to as "compound A") is a compound represented by the following chemical structural formula. Compound A or a pharmaceutically acceptable salt thereof is known to be useful as an active ingredient in a pharmaceutical composition for the treatment of cancer (Patent literature 1, Patent literature 2, Patent literature 3, and Patent literature 4).

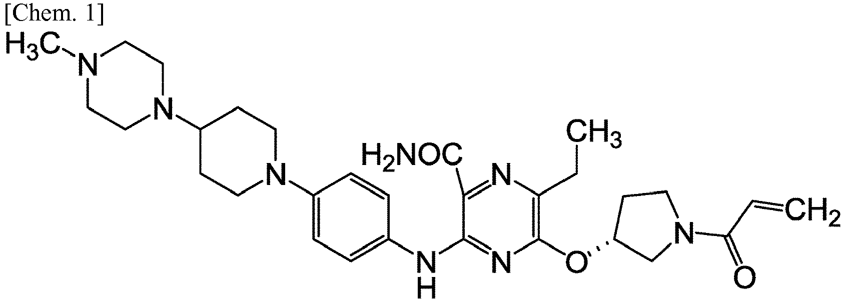

As compound A or a pharmaceutically acceptable salt thereof, Patent literature 1 discloses its free form in Example 54, and its monomethanesulfonate in Example 261, and discloses that the inhibitory action on an epidermal growth factor receptor (EGFR) mutant kinase has been confirmed.

Even today, in which the advancement of medical care is remarkable, especially for cancer, treatment satisfaction is low, and the contribution of further medicines is required. Providing stable medicines to medical professionals and people requiring treatment of illness plays an important role in the contribution to the health of people worldwide.

There are various destabilization mechanisms of drugs. There is a problem with the stability of the drug itself; in a pharmaceutical composition, particularly in a solid pharmaceutical composition, for example, there is a problem between the interaction of a drug and various pharmaceutical additives, or there are causes of instability of a drug in the manufacturing process; in a pharmaceutical composition, a drug reacts with moisture contained in pharmaceutical additives or the like (for example, Patent literature 5); and the like. As described above, in terms of the nature of pharmaceuticals, it is extremely important to inhibit the generation of related substances, or the increase in the amount of related substances. However, a general method has not been established for stabilization of drugs, and even at present, it is sought for stabilization in a manner suitable for each drug.

### CITATION LIST

### PATENT LITERATURE

[Patent literature 1] WO 2013/108754
[Patent literature 2] WO 2015/182628
[Patent literature 3] WO 2016/175252
[Patent literature 4] WO2017/090699
[Patent literature 5] Japanese Unexamined Patent Publication (Kokai) No. 10-147524 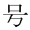

### SUMMARY OF INVENTION

### TECHNICAL PROBLEM

A problem of the present invention is to provide a stable pharmaceutical composition comprising compound A or a pharmaceutically acceptable salt thereof.

### SOLUTION TO PROBLEM

Despite the fact that compound A monomethanesulfonate is itself stable, the inventors found that related substances and the amount of the related substances increased over time when a pharmaceutical composition containing the drug together with pharmaceutical additives was prepared in a conventional method, or via various formulation steps, and stored under severe conditions. Further, the inventors found that, when the measurement was carried out by "(Test for related substance)" described in Experimental Example 1 below, a related substance detected at a relative retention time to compound A of about 1.42 (hereinafter sometimes referred to as "related substance A") was the main related substance of compound A, and was a dimer of compound A; and the generation of the dimer was promoted by moisture contained in the pharmaceutical additives in the pharmaceutical composition; and the like.

The dimer of compound A is represented by the following chemical structural formula.

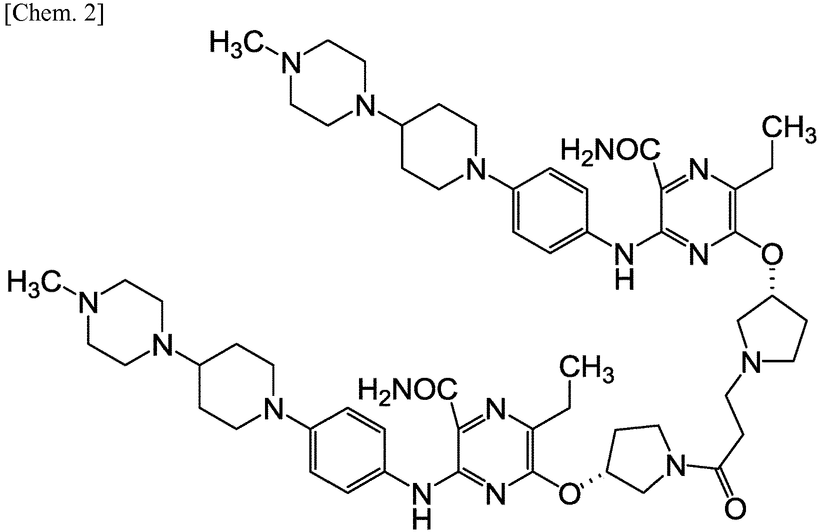

Further, the inventors found that, along with the increase of the dimer of compound A, another related substance detected at a relative retention time to compound A of about 1.58 (hereinafter sometimes referred to as "related substance B") also increased.

With respect to a stable pharmaceutical composition comprising compound A or a pharmaceutically acceptable salt thereof, the applicant has filed an international application No. PCT/JP2016/063004. Examples of the application disclose stable pharmaceutical compositions (capsules) prepared by a production method comprising steps of pulverization of compound A or a pharmaceutically acceptable salt thereof, mixing, capsule filling, and the like. In contrast, the present invention relates to stabilization when preparing a pharmaceutical composition using water, while also desiring development of tableting. For example, wet granulation, which is one of granulation methods, is a method of making particles by spraying and moistening powder with water, or a solution in which a binder is dissolved, and drying the moisture. While improving fluidity and tabletability, in the case of a drug in which the generation of related substances is promoted due to moisture, such as compound A, further stabilization techniques are desired for preparing a pharmaceutical composition using water.

Under these circumstances, the inventors focused attention on the stability of compound A monomethanesulfonate, and conducted intensive studies to complete the present invention.

The present invention relates to:
[1] a pharmaceutical composition comprising:
   (1) 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide or a pharmaceutically acceptable salt thereof, and
   (2) potassium chloride and/or sodium chloride,
[2] the pharmaceutical composition of [1], wherein the content of potassium chloride and/or sodium chloride is 0.5% by weight to 80% by weight with respect to the weight of 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide or a pharmaceutically acceptable salt thereof,
[3] the pharmaceutical composition of [1] or [2], wherein the content of potassium chloride and/or sodium chloride is 0.1% by weight to 85% by weight with respect to the weight of the pharmaceutical composition,
[4] the pharmaceutical composition of any one of [1] to [3], wherein the pharmaceutical composition is a tablet,
[5] a method of producing a pharmaceutical composition, characterized by mixing
   (1) 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide or a pharmaceutically acceptable salt thereof, and
   (2) potassium chloride and/or sodium chloride; and
   compression-molding the mixture,
[6] the method of producing a pharmaceutical composition of [5], wherein the content of potassium chloride and/or sodium chloride is 0.5% by weight to 80% by weight with respect to the weight of 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide or a pharmaceutically acceptable salt thereof,
[7] the method of producing a pharmaceutical composition of [5] or [6], wherein the content of potassium chloride and/or sodium chloride is 0.1 % by weight to 85% by weight with respect to the weight of the pharmaceutical composition,
[8] a method of producing a pharmaceutical composition comprising:
   (a) mixing (1) 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide or a pharmaceutically acceptable salt thereof, (2) potassium chloride and/or sodium chloride, and (3) a pharmaceutical additive;
   (b) preparing a binder liquid by dissolving or dispersing potassium chloride and/or sodium chloride in a solvent; and
   (c) mixing the mixture prepared in (a) with the binder liquid prepared in (b), and performing granulation,
[9] a method of producing a pharmaceutical composition comprising:
   (a) mixing (1) 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide or a pharmaceutically acceptable salt thereof, (2) potassium chloride and/or sodium chloride, and (3) a pharmaceutical additive;
   (b) preparing a binder liquid using a solvent; and
   (c) mixing the mixture prepared in (a) with the binder liquid prepared in (b), and performing granulation,
[10] a method of producing a pharmaceutical composition comprising:
   (a) mixing (1) 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide or a pharmaceutically acceptable salt thereof, (2) potassium chloride and/or sodium chloride, and (3) a pharmaceutical additive;
   (b) preparing a binder liquid by dissolving or dispersing potassium chloride and/or sodium chloride in a liquid containing a pharmaceutical additive; and
   (c) mixing the mixture prepared in (a) with the binder liquid prepared in (b), and performing granulation, and
[11] a method of stabilizing a pharmaceutical composition comprising 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide or a pharmaceutically acceptable salt thereof, said method using potassium chloride and/or sodium chloride.

### ADVANTAGEOUS EFFECTS OF INVENTION

According to the present invention, a stable pharmaceutical composition comprising compound A or a pharmaceutically acceptable salt thereof, for example, a pharmaceutical composition that is stable against humidity, moisture, or temperature, can be provided.

### DESCRIPTION OF EMBODIMENTS

The term "stable" or "stabilization of a pharmaceutical composition" as used herein means a state where compound A or a pharmaceutically acceptable salt thereof is stabilized in a pharmaceutical composition (formulation) including a pharmaceutical additive and compound A or a pharmaceutically acceptable salt thereof. The state can be evaluated, for example, by calculating the amount (percentage, %) or the like of related substances over time, in comparison with that at the beginning of the test. As the index, it is defined as a stable state sufficient to provide it as a pharmaceutical composition in the medical field.

For example, with respect to the percentage of related substances, in which a change is observed due to the moisture contained in a pharmaceutical composition, for example, the pharmaceutical composition is allowed to stand under storage conditions of 40°C and 75% relative humidity (hereinafter sometimes abbreviated as %RH) (opened, closed, or sealed) for 1 month, and the percentage of the related substances is measured by a high performance liquid chromatographic method (hereinafter sometimes abbreviated as an HPLC method). The related substances are measured, for example, by "Test for related substance" described in Experimental Example 1 below. The content of related substance A or related substance B is calculated by measuring the peak area of each related substance contained in the pharmaceutical composition by the HPLC method, and dividing the peak area of related substance A or the peak area of related substance B by the peak area of compound A.

The phrase "to improve the stability of compound A or a pharmaceutically acceptable salt thereof' as used herein means that, when the pharmaceutical composition containing compound A or a pharmaceutically acceptable salt thereof is stored, "the generation of related substances of compound A or a pharmaceutically acceptable salt thereof during storage is inhibited".

With respect to conditions for a stability test, instead of the above-mentioned conditions of 40°C and 75%RH (opened, closed, or sealed) for 1 month, the same conditions except that the storage period is 2 months, 3 months, or 6 months can be used. Further, the conditions of 25°C and 60%RH (opened, closed, or sealed) can be appropriately combined with a storage period selected from 1 month to 24 months, or to 36 months. Furthermore, in order to evaluate for a short period of time, for example, the conditions of 70°C for 9 days (opened, closed, or sealed conditions, such as aluminum-aluminum (Al-Al)-packaging and closed conditions) can be used. In this case, with respect to evaluation of "to be stable" as used herein, for example, a method that is judged to be scientifically valid, such as an extrapolation method, may be used, so that the conditions thermodynamically correspond to the result under storage conditions at 40°C for 6 months.

The related substances in which a change is observed due to moisture are defined, for example, as related substance A or related substance B under the measurement conditions of the HPLC method described in "Test for related substance" described in Experimental Example 1 below. Stability evaluation can be carried out by an absolute evaluation, in which the amounts of the related substances are evaluated over time, or a relative evaluation, in which the amounts of the related substances at the beginning of the test is compared with that at the time of the measurement. "To be stable" means that the increased amount of related substance A after 1 month, 2 months, or 3 months from the beginning of the test for a relative evaluation is 0.40% or less, and that of related substance B is less than 0.05%.

The phrase "inhibition of the generation of the related substances", "inhibition of the increase in the amount of the related substances", or "improvement of stability" can be regarded as the same meaning as the above "stable" state.

The pharmaceutical composition of the present invention will be explained below.

Compound A or a pharmaceutically acceptable salt thereof, which is used in the present invention, is easily available, for example, by a method described in Patent literature 1, or in a similar fashion to that.

Compound A may be in a free form in an embodiment, and may form a pharmaceutically acceptable salt with an acid in other embodiments. Examples of such a salt include an acid addition salt with an inorganic acid, such as hydrochloric acid, hydrobromic acid, hydroiodic acid, sulfuric acid, nitric acid, phosphoric acid, or the like; and an acid addition salt with an organic acid, such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid (mesylic acid), ethanesulfonic acid, benzenesulfonic acid, p-toluenesulfonic acid, aspartic acid, glutamic acid, or the like. "Compound A or a pharmaceutically acceptable salt" includes solvates of compound A, in particular, such as hydrates or ethanol solvates, as well as solvates of an acid addition salt of compound A. In an embodiment, it is 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide monomethanesulfonate.

These salts can be prepared by conventional methods.

For normal oral administration, the daily dosage is suitably about 0.001 to 100 mg/kg in an embodiment, 0.1 to 30 mg/kg in another embodiment, and 0.1 to 10 mg/kg in still another embodiment, and this is administered in one dosage, or divided into 2 to 4 daily dosages. The dosage may be appropriately determined according to individual cases with consideration of symptoms, age, sex, and the like.

The content of compound A or a pharmaceutically acceptable salt thereof is, for example, per pharmaceutical composition, about 0.1% by weight or more and about 99.9% by weight or less in an embodiment, about 4% by weight or more and about 60% by weight or less in an embodiment, about 4% by weight or more and about 55% by weight or less in an embodiment, and about 4% by weight or more and about 50% by weight or less in an embodiment.

Potassium chloride and/or sodium chloride, which are used in the present invention, are not particularly limited, so long as they are potassium chloride and/or sodium chloride that are acceptable as pharmaceutical additives.

The content of potassium chloride and/or sodium chloride is not particularly limited, so long as the generation of related substances of compound A or a pharmaceutically acceptable salt thereof contained in the pharmaceutical composition can be inhibited. More particularly, for example, the content with respect to the weight of compound A or a pharmaceutically acceptable salt thereof is 0.5% by weight to 80% by weight in an embodiment, 2% by weight to 55% by weight in an embodiment, and 5% by weight to 35% by weight in an embodiment. Further, the content in the pharmaceutical composition is 0.1% by weight to 85% by weight in an embodiment, 0.5% by weight to 30% by weight in an embodiment, 2% by weight to 15% by weight in an embodiment, and 4% by weight to 15% by weight in an embodiment.

The pharmaceutical composition of the present invention may be various pharmaceutical compositions, such as tablets, capsules, granules, powder, fine granules, and the like. In an embodiment, it may be tablets.

In the pharmaceutical composition of the present invention, it may be formulated by appropriately using various pharmaceutical additives to the extent that the desired effects of the present invention can be achieved. Such pharmaceutical additives are not particularly limited, so long as they are pharmaceutically acceptable and pharmacologically acceptable. Examples of the pharmaceutical additives include fillers, corrigents, binders, disintegrating agents, effervescent agents, lubricants, fluidizing agents, sweeteners, flavors, buffers, antioxidants, surfactants, and the like.

Examples of the fillers include lactose monohydrate (USP-NF)(lactose hydrate, JP), anhydrous dibasic calcium phosphate, sucrose, D-mannitol, D-sorbitol, starch, pregelatinized starch, dextran, dextrin, crystalline cellulose, corn starch, calcium carbonate, low substituted hydroxypropyl cellulose, carmellose sodium, gum arabic, pullulan, light anhydrous silicic acid, synthetic aluminum silicate, magnesium metasilicate aluminate, and the like. In an embodiment, it may be dextran, dextrin, crystalline cellulose, corn starch, calcium carbonate, lactose monohydrate (USP-NF)(lactose hydrate, JP), anhydrous dibasic calcium phosphate, or D-mannitol.

Examples of the corrigents include citric acid, tartaric acid, malic acid, and the like.

Examples of the binders include hydroxypropyl cellulose, and the like.

Examples of the disintegrating agents include low substituted hydroxypropyl cellulose, croscarmellose sodium, and the like.

Examples of the effervescent agents include sodium bicarbonate, and the like.

Examples of the lubricants include magnesium stearate, calcium stearate, sodium stearyl fumarate, and the like.

Examples of the fluidizing agents include light anhydrous silicic acid, and the like.

Examples of the sweeteners include saccharin sodium, dipotassium glycyrrhizinate, aspartame, stevia, thaumatin, and the like.

Examples of the flavors include lemon, lemon-lime, orange, menthol, and the like.

Examples of the buffers include citric acid, succinic acid, fumaric acid, tartaric acid, ascorbic acid, and salts thereof; glutamic acid, glutamine, glycine, aspartic acid, alanine, arginine, and salts thereof; magnesium oxide, zinc oxide, magnesium hydroxide, phosphoric acid, boric acid, and salts thereof; and the like.

Examples of the antioxidants include ascorbic acid, dibutylhydroxytoluene, propyl gallate, and the like.

Examples of the surfactants include polysorbate 80, sodium lauryl sulfate, polyoxyethylene hydrogenated castor oil, and the like.

These pharmaceutical additives may be used alone, or in a combination of two or more.

With respect to the contents of the pharmaceutical additives, each pharmaceutical additive may be used in an amount such that the desired effects of the present invention may be achieved.

The method of producing the pharmaceutical composition of the present invention will be explained.

The pharmaceutical composition of the present invention can be prepared by methods known per se.

More particularly, the method includes various formulation steps, such as de-lumping of compound A or a pharmaceutically acceptable salt thereof, mixing, granulation, drying, molding (tableting), film-coating, packaging, and the like.

The method of producing the pharmaceutical composition of the present invention will be explained below.

### De-lumping step

In the de-lumping step, both the apparatus and the means are not particularly limited, so long as it is a method in which the drug and/or appropriate pharmaceutical additives can be de-lumped in a normal pharmaceutical manner. Examples of an equipment for de-lumping include a hammer mill, a ball mill, a comil, a colloid mill, and the like. The conditions for de-lumping may be appropriately selected and are not particularly limited.

### Mixing step

A method of mixing compound A or a pharmaceutically acceptable salt thereof, potassium chloride and/or sodium chloride, and pharmaceutical additives; a method of mixing a granulated product obtained in the granulation step below and pharmaceutical additives; or a method of a granulated product obtained in the granulation step below, potassium chloride and/or sodium chloride, and pharmaceutical additives; is not particularly limited, so long as it is a method in which each component can be uniformly mixed in a normal pharmaceutical manner. Examples of an apparatus include a fluidized bed mixer, a V-type blender, a ribbon blender, a container mixer, a high shear mixer, and the like.

### Granulation step

A method of granulating the mixture is not particularly limited, so long as it is a method in which the mixture can be granulated in a normal pharmaceutical manner. Examples of an apparatus include a fluidized bed granulator, a melting and agitation granulator, a high shear granulator, a granulator with de-lumping (pulverization), an extrusion granulator, a tumbling fluidized bed granulator, a spray granulator, a dry granulator, a twin-screw extruder, and the like. In an embodiment, it is a fluidized bed granulator or a high shear granulator, and in an embodiment, it is a fluidized bed granulator.

Examples of an embodiment in which potassium chloride and/or sodium chloride are added in the granulation step include (1) an embodiment in which a binder liquid is prepared by dissolving or dispersing potassium chloride and/or sodium chloride in a solvent, such as purified water, ethanol, methanol, or the like, and compound A or a pharmaceutically acceptable salt thereof and/or pharmaceutical additives are sprayed with the binder liquid to granulate the mixture; (2) an embodiment in which potassium chloride and/or sodium chloride are charged into a granulator as powdered state, and granulated together with compound A or a pharmaceutically acceptable salt thereof and/or pharmaceutical additives, while adding a solvent, such as purified water, ethanol, methanol, or the like, prepared as a binder liquid; and (3) an embodiment in which a binder liquid is prepared by dissolving or dispersing potassium chloride and/or sodium chloride in a liquid containing pharmaceutical additives, such as a binder or the like, and compound A or a pharmaceutically acceptable salt thereof and/or pharmaceutical additives are granulated with the binder liquid. A more stable pharmaceutical composition can be obtained by granulating using a binder prepared by dissolving or dispersing potassium chloride and/or sodium chloride in a solvent.

A still more stable pharmaceutical composition can be obtained by controlling the water content of the granulated product during granulation to be low.

### Drying step

A method of drying the granulated product obtained in the granulation step is not particularly limited, so long as it is a method in which the granulated product can be dried in a normal pharmaceutical manner. Examples of an apparatus include a forced-air dryer, a dryer under reduced pressure, a vacuum dryer, a fluidized bed dryer, and the like.

After drying, the dried product may be sieved and sized using a sieve, a comil, or the like, if desired.

### Molding (tableting) step

A molding method is not particularly limited, so long as it is a method by which molding can be carried out in a normal pharmaceutical manner. Examples of an apparatus include a rotary tableting machine, a single punch tableting machine, an oil press, and the like.

In the molding step, for example, a method in which a mixture or a granulated product containing compound A or a pharmaceutically acceptable salt thereof and potassium chloride and/or sodium chloride, or a mixed product (a mixed product before compression-molding, in particular, a mixed product before tableting) prepared by mixing a granulated product with potassium chloride and/or sodium chloride and various pharmaceutical additives such as a lubricant is compression-molded to form tablets; a direct tableting method in which compound A or a pharmaceutically acceptable salt thereof, potassium chloride and/or sodium chloride, and appropriate pharmaceutical additives are mixed and compression-molded to obtain tablets; or the like, may be used.

### Film-coating step

A method of film-coating the tablets prepared in the molding step with a film-coating agent is not particularly limited, so long as it is a method in which film-coating can be carried out in a normal pharmaceutical manner.

Examples of an apparatus include a pan coating machine, a fluidized bed coating machine, and the like. As the film-coating agent, hypromellose, talc, polyethylene glycol 8000 (USP-NF)(Macrogol 6000, JP), titanium dioxide (USP-NF)(titanium oxide, JP), ferric oxide (yellow)(USP-NF)(yellow ferric oxide, JP), ferric oxide (red)(USP-NF)(red ferric oxide, JP), and the like, may be used.

When film-coating is carried out, it is preferable to carry out the film-coating under conditions such that the surface of the tablet does not become wet and the film-coating liquid becomes easy to dry.

The present invention includes a method of stabilizing a pharmaceutical composition comprising compound A or a pharmaceutically acceptable salt thereof, said method using potassium chloride and/or sodium chloride.

With respect to "compound A or a pharmaceutically acceptable salt thereof' and "potassium chloride and/or sodium chloride", which are used in the stabilizing method of the present invention, the explanations therefor described in the pharmaceutical composition of the present invention can be directly applied.

In the stabilizing method of the present invention, when the pharmaceutical composition comprising compound A or a pharmaceutically acceptable salt thereof is produced, the generation of related substances (in particular, related substances in which a change is observed due to the moisture contained in the pharmaceutical composition) can be inhibited by containing potassium chloride and/or sodium chloride.

With respect to the content of each component, their blending method, and the like in the stabilizing method, the explanations therefor described in the pharmaceutical composition of the present invention can be directly applied.

### EXAMPLES

The present invention will now be further illustrated by, but is by no means limited to, the following Examples and Experimental Examples.

Compound A monomethanesulfonate, which was used in the Examples below, had been prepared in accordance with a method described in WO 2013/108754.

### (Example 1)

A fluidized bed granulator was used to mix 117.1 parts of de-lumped compound A monomethanesulfonate, 77.3 parts of lactose monohydrate (USP-NF)(lactose hydrate, JP), and 14.4 parts of potassium chloride (manufactured by MERCK; unless otherwise stated, the same compound was used in the following). A binder liquid, which had a solid content of 14% by weight, was prepared by dissolving 9.6 parts of potassium chloride and 7.2 parts of hydroxypropyl cellulose in water. The mixture was granulated by spraying the binder liquid, and dried to obtain a granulated product.

To 225.6 parts of the granulated product obtained, 24.0 parts of low substituted hydroxypropyl cellulose and 2.4 parts of magnesium stearate were added, and the mixture was mixed using a mixer to obtain a mixed product before tableting.

The obtained mixed product before tableting was formed into tablets, using a rotary tableting machine, to obtain tablets (uncoated tablets).

The obtained uncoated tablets were film-coated using a film-coating machine to obtain tablets (film-coated tablets). In connection with this, the film-coating agent contained hypromellose, talc, polyethylene glycol 8000 (Macrogol 6000 JP), titanium dioxide (USP-NF)(titanium oxide, JP), ferric oxide (yellow)(USP-NF)(yellow ferric oxide, JP), and ferric oxide (red)(USP-NF)(red ferric oxide, JP) (the same film-coating agent was used in the following).

### (Example 2)

A fluidized bed granulator was used to mix 117.1 parts of de-lumped compound A monomethanesulfonate, 77.6 parts of lactose monohydrate (USP-NF)(lactose hydrate, JP), and 10.56 parts of potassium chloride. A binder liquid, which had a solid content of 15.2% by weight, was prepared by dissolving 5.94 parts of potassium chloride and 6.6 parts of hydroxypropyl cellulose in water. The mixture was granulated by spraying the binder liquid, and dried to obtain a granulated product.

To 217.8 parts of the granulated product obtained, 2.2 parts of magnesium stearate was added, and the mixture was mixed using a mixer to obtain a mixed product before tableting.

The obtained mixed product before tableting was formed into tablets to obtain tablets (uncoated tablets).

The obtained uncoated tablets were film-coated using a film-coating machine to obtain tablets (film-coated tablets).

### (Comparative Example 1)

A fluidized bed granulator was used to mix 117.1 parts of de-lumped compound A monomethanesulfonate and 74.9 parts of lactose monohydrate (USP-NF)(lactose hydrate, JP). A binder liquid, which had a solid content of 8% by weight, was prepared by dissolving 6.0 parts of hydroxypropyl cellulose in water. The mixture was granulated by spraying the binder liquid, and dried to obtain a granulated product.

To 198.0 parts of the granulated product obtained, 2.0 parts of magnesium stearate was added, and the mixture was mixed using a mixer to obtain a mixed product before tableting.

The obtained mixed product before tableting was formed into tablets, using a rotary tableting machine, to obtain tablets (uncoated tablets).

The obtained uncoated tablets were film-coated using a film-coating machine to obtain tablets (film-coated tablets).

### (Comparative Example 2)

A fluidized bed granulator was used to mix 117.1 parts of de-lumped compound A monomethanesulfonate and 101.3 parts of lactose monohydrate (USP-NF)(lactose hydrate, JP). A binder liquid, which had a solid content of 6% by weight, was prepared by dissolving 7.2 parts of hydroxypropyl cellulose in water. The mixture was granulated by spraying the binder liquid, and dried to obtain a granulated product.

To 225.6 parts of the granulated product obtained, 2.4 parts of magnesium stearate was added, and the mixture was mixed using a mixer to obtain a mixed product before tableting.

The obtained mixed product before tableting was formed into tablets, using a rotary tableting machine, to obtain tablets (uncoated tablets).

The obtained uncoated tablets were film-coated using a film-coating machine to obtain tablets (film-coated tablets).

**[Table 1]**

| | Example 1 | Example 2 | Comparative Example 1 | Comparative Example 2 |
|---|---|---|---|---|
| Compound A monomethanesulfonate | 117.1 | 117.1 | 117.1 | 117.1 |
| Lactose monohydrate (USP-NF) (Lactose hydrate, JP) | 77.3 | 77.6 | 74.9 | 101.3 |
| Potassium chloride | 24.0 | 16.5 | - | - |
| Hydroxypropyl cellulose | 7.2 | 6.6 | 6.0 | 7.2 |
| Low substituted hydroxypropyl cellulose | 24.0 | - | - | - |
| Magnesium stearate | 2.4 | 2.2 | 2.0 | 2.4 |
| Uncoated tablet | 252.0 | 220.0 | 200.0 | 228.0 |
| Film coating agent | 8.0 | 6.6 | 6.0 | 8.0 |
| Film-coated tablet | 260.0 | 226.6 | 206.0 | 236.0 |

| | | | | |
|---|---|---|---|---|
| (Unit: mg) | | | | |

### (Example 3)

A fluidized bed granulator was used to mix 117.1 parts of de-lumped compound A monomethanesulfonate, 83.3 parts of lactose monohydrate (USP-NF)(lactose hydrate, JP), and 0.6 parts of potassium chloride. A binder liquid, which had a solid content of 14% by weight, was prepared by dissolving 5.4 parts of potassium chloride and 7.2 parts of hydroxypropyl cellulose in water. The mixture was granulated by spraying the binder liquid, and dried to obtain a granulated product.

To 213.6 parts of the granulated product obtained, 24.0 parts of low substituted hydroxypropyl cellulose and 2.4 parts of magnesium stearate were added, and the mixture was mixed using a mixer to obtain a mixed product before tableting.

The obtained mixed product before tableting was formed into tablets, using a rotary tableting machine, to obtain tablets (uncoated tablets).

The obtained uncoated tablets were film-coated using a film-coating machine to obtain tablets (film-coated tablets).

### (Example 4)

A fluidized bed granulator was used to mix 117.1 parts of de-lumped compound A monomethanesulfonate, 65.3 parts of lactose hydrate, and 18.6 parts of potassium chloride. A binder liquid, which had a solid content of 14% by weight, was prepared by dissolving 5.4 parts of potassium chloride and 7.2 parts of hydroxypropyl cellulose in water. The mixture was granulated by spraying the binder liquid, and dried to obtain a granulated product.

To 213.6 parts of the granulated product obtained, 24.0 parts of low substituted hydroxypropyl cellulose and 2.4 parts of magnesium stearate were added, and the mixture was mixed using a mixer to obtain a mixed product before tableting.

The obtained mixed product before tableting was formed into tablets, using a rotary tableting machine, to obtain tablets (uncoated tablets).

The obtained uncoated tablets were film-coated using a film-coating machine to obtain tablets (film-coated tablets).

**[Table 2]**

| | Example 3 | Example 4 |
|---|---|---|
| Compound A monomethanesulfonate | 117.1 | 117.1 |
| Lactose monohydrate (USP-NF) (Lactose hydrate, JP) | 83.3 | 65.3 |
| Potassium chloride | 6.0 | 24.0 |
| Hydroxypropyl cellulose | 7.2 | 7.2 |
| Low substituted hydroxypropyl cellulose | 24.0 | 24.0 |
| Magnesium stearate | 2.4 | 2.4 |
| Uncoated tablet | 240.0 | 240.0 |
| Film coating agent | 9.6 | 9.6 |
| Film-coated tablet | 249.6 | 249.6 |

| | | |
|---|---|---|
| (Unit: mg) | | |

### (Example 5)

A fluidized bed granulator was used to mix 117.1 parts of de-lumped compound A monomethanesulfonate and 101.3 parts of lactose hydrate. A binder liquid, which had a solid content of 6% by weight, was prepared by dissolving 7.2 parts of hydroxypropyl cellulose in water. The mixture was granulated by spraying the binder liquid, and dried to obtain a granulated product.

To 225.6 parts of the granulated product obtained, 10.6 parts of potassium chloride, 26.7 parts of low substituted hydroxypropyl cellulose, and 2.4 parts of magnesium stearate were added, and the mixed product was formed into tablets to obtain tablets (uncoated tablets).

### (Example 6)

A fluidized bed granulator was used to mix 117.1 parts of de-lumped compound A monomethanesulfonate and 101.3 parts of lactose hydrate. A binder liquid, which had a solid content of 6% by weight, was prepared by dissolving 7.2 parts of hydroxypropyl cellulose in water. The mixture was granulated by spraying the binder liquid, and dried to obtain a granulated product.

To 225.6 parts of the granulated product obtained, 40.1 parts of potassium chloride, 26.7 parts of low substituted hydroxypropyl cellulose, and 2.4 parts of magnesium stearate were added, and the mixed product was formed into tablets to obtain tablets (uncoated tablets).

**[Table 3]**

| | Example 5 | Example 6 |
|---|---|---|
| Compound A monomethanesulfonate | 117.1 | 117.1 |
| Lactose monohydrate (USP-NF) (Lactose hydrate, JP) | 101.3 | 101.3 |
| Hydroxypropyl cellulose | 7.2 | 7.2 |
| Potassium chloride | 10.6 | 40.1 |
| Low substituted hydroxypropyl cellulose | 26.7 | 26.7 |
| Magnesium stearate | 2.4 | 2.4 |
| Uncoated tablet | 265.3 | 294.8 |

| | | |
|---|---|---|
| (Unit: mg) | | |

### (Example 7)

A fluidized bed granulator was used to mix 117.1 parts of de-lumped compound A monomethanesulfonate, 77.6 parts of lactose hydrate, and 10.56 parts of sodium chloride (manufactured by MERCK; unless otherwise stated, the same compound was used in the following). A binder liquid, which had a solid content of 15.2% by weight, was prepared by dissolving 5.94 parts of sodium chloride and 6.6 parts of hydroxypropyl cellulose in water. The mixture was granulated by spraying the binder liquid, and dried to obtain a granulated product.

To 217.8 parts of the granulated product obtained, 2.2 parts of magnesium stearate was added, and the mixture was mixed using a mixer to obtain a mixed product before tableting.

The obtained mixed product before tableting was formed into tablets to obtain tablets (uncoated tablets).

The obtained uncoated tablets were film-coated using a film-coating machine to obtain tablets (film-coated tablets).

**[Table 4]**

| | Example 7 |
|---|---|
| Compound A monomethanesulfonate | 117.1 |
| Lactose monohydrate (USP-NF) (Lactose hydrate, JP) | 77.6 |
| Sodium chloride | 16.5 |
| Hydroxypropyl cellulose | 6.6 |
| Magnesium stearate | 2.2 |
| Uncoated tablet | 220.0 |
| Film coating agent | 6.6 |
| Film-coated tablet | 226.6 |

| | |
|---|---|
| (Unit: mg) | |

### (Experimental Example 1: Stability test)

After the tablets obtained in Examples 1 to 4 and 7 and Comparative Examples 1 to 2 were allowed to stand in a packaged form of aluminum-aluminum (Al-Al)-packaging under storage conditions of 40°C and 75%RH for 3 months, the amounts of related substances (compound A at a relative retention time of about 1.42, and compound B at a relative retention time of about 1.58) were measured by the test for related substance below to evaluate the stability over time, in comparison with those at the beginning of the test, in accordance with a relative evaluation in which increase amounts were calculated by subtracting the amounts of the related substances at the beginning of the test from those at 1 month, 2 months, and 3 months. The results are shown in Tables 5 and 6.

### (Test for related substance)

The amounts of generated related substances were measured by a high performance liquid chromatographic method (an HPLC method). The amount of a related substance is calculated by measuring the peak area of each related substance contained in a pharmaceutical composition by the HPLC method, and dividing the peak area of related substance A or the peak area of related substance B by the peak area of compound A.
- Measurement wavelength: 210 nm
- Column: CAPCELL PAK C18 AQ (4.6 mm × 250 mm, 3 µm)
- Column temperature: a constant temperature around 45°C
- Mobile phase: A mixed solution of perchloric acid aqueous solution and acetonitrile and methanol
- Flow rate: about 1.2 mL/min
- Injection amount: 10 µL

**[Table 5]**

| Related substance A (Increased amount, %) | 1 month | 2 months | 3 months |
|---|---|---|---|
| Example 1 | 0.32 | 0.35 | 0.38 |
| Example 2 | 0.14 | 0.28 | 0.28 |
| Example 3 | 0.11 | 0.23 | 0.23 |
| Example 4 | 0.23 | 0.24 | 0.35 |
| Example 7 | 0.13 | Not measured | Not measured |
| Comparative Example 1 | 1.04 | 1.51 | 1.64 |
| Comparative Example 2 | 0.45 | 0.54 | 0.59 |

**[Table 6]**

| Related substance B (Increased amount, %) | 1 month | 2 months | 3 months |
|---|---|---|---|
| Example 1 | 0.02 | 0.03 | 0.05 |
| Example 2 | Not detected | 0.03 | 0.05 |
| Example 3 | Not detected | 0.03 | 0.02 |
| Example 4 | 0.02 | 0.02 | 0.04 |
| Example 7 | 0.02 | Not measured | Not measured |
| Comparative Example 1 | 0.08 | 0.21 | 0.33 |
| Comparative Example 2 | 0.05 | 0.07 | 0.11 |

### (Experimental Example 2: Dissolution test)

With respect to the film-coated tablets obtained in Examples 1 to 4 and the uncoated tablets obtained in Examples 5 and 6, the dissolution rate was measured in accordance with a Dissolution Test, a paddle method (50 rpm) of the Japanese Pharmacopoeia. As a test fluid, 900 mL of 0.1 N hydrochloric acid was used. The measurement was carried out by ultraviolet-visible spectrophotometry (UV method (measurement wavelength: 306 nm)). The results are shown in Table 7.

The tablets of Examples 1 to 6 showed rapid dissolution.

**[Table 7]**

| Dissolution rate (%) | Value in 5 minutes | Value in 15 minutes | Value in 30 minutes |
|---|---|---|---|
| Example 1 | 70.3 | 106.1 | 106.1 |
| Example 2 | 29.5 | 81.7 | 103.1 |
| Example 3 | 15.8 | 52.3 | 87.2 |
| Example 4 | 29.9 | 100.8 | 105.1 |
| Example 5 | 29.9 | 90.0 | 97.1 |
| Example 6 | 39.0 | 102.6 | 102.6 |

### INDUSTRIAL APPLICABILITY

The present invention is useful as a formulation technique for providing a stable pharmaceutical composition comprising compound A or a pharmaceutically acceptable salt thereof (for example, compound A monomethanesulfonate).

Although the present invention has been described with reference to specific embodiments, various changes and modifications obvious to those skilled in the art are possible without departing from the scope of the appended claims.

## Claims

1. A pharmaceutical composition comprising:
(1) 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide or a pharmaceutically acceptable salt thereof, and
(2) potassium chloride and/or sodium chloride.

2. The pharmaceutical composition according to claim 1, wherein the content of potassium chloride and/or sodium chloride is 0.5% by weight to 80% by weight with respect to the weight of 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide or a pharmaceutically acceptable salt thereof.

3. The pharmaceutical composition according to claim 1 or 2, wherein the content of potassium chloride and/or sodium chloride is 0.1% by weight to 85% by weight with respect to the weight of the pharmaceutical composition.

4. The pharmaceutical composition according to any one of claims 1 to 3, wherein the pharmaceutical composition is a tablet.

5. A method of producing a pharmaceutical composition, **characterized by** mixing
(1) 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide or a pharmaceutically acceptable salt thereof, and
(2) potassium chloride and/or sodium chloride; and
compression-molding the mixture.

6. The method of producing a pharmaceutical composition according to claim 5, wherein the content of potassium chloride and/or sodium chloride is 0.5% by weight to 80% by weight with respect to the weight of 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide or a pharmaceutically acceptable salt thereof.

7. The method of producing a pharmaceutical composition according to claim 5 or 6, wherein the content of potassium chloride and/or sodium chloride is 0.1% by weight to 85% by weight with respect to the weight of the pharmaceutical composition.

8. A method of producing a pharmaceutical composition comprising:
(a) mixing (1) 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide or a pharmaceutically acceptable salt thereof, (2) potassium chloride and/or sodium chloride, and (3) a pharmaceutical additive;
(b) preparing a binder liquid by dissolving or dispersing potassium chloride and/or sodium chloride in a solvent; and
(c) mixing the mixture prepared in (a) with the binder liquid prepared in (b), and performing granulation.

9. A method of stabilizing a pharmaceutical composition comprising 5-{[(3R)-1-acryloylpyrrolidin-3-yl]oxy}-6-ethyl-3-({4-[4-(4-methylpiperazin-1-yl)piperidin-1-yl]phenyl}amino)-pyrazine-2-carboxamide or a pharmaceutically acceptable salt thereof, said method using potassium chloride and/or sodium chloride.
